# EUROPEAN PATENT APPLICATION

(11) **EP 2 123 667 A1**
(43) Date of publication of application: **25.11.2009**
(21) Application number: 08720933.4
(22) Date of filing: 27.02.2008
(51) Int. Cl.: C07K 7/64, A61K 47/42, A61K 47/48, G01T 1/161

(54) **BRAIN-MIGRATING POLYPEPTIDE HAVING POLYVALENT BOND AND IMPROVED METABOLIC STABILITY**

(30) Priority: 05.03.2007 JP 2007054260
(71) Applicant: Proteus Sciences Co., Ltd., Nagoya-shi Aichi 464-0858 (JP); National Institute of Radiological Sciences, Chiba-shi, Chiba 263-8555 (JP)
(72) Inventor: NAKAJO, Tomohiro, Fujimino-shi Saitama 356-0029 (JP); HARA, Hirotaka, Konan-shi Aichi 483-8423 (JP); YAMAMOTO, Kazumasa, Nagoya-shi Aichi 458-0813 (JP); SUZUKI, Hiromi, Toyota-shi Aichi 470-1201 (JP); SAWADA, Makoto, Kasugai-shi Aichi 487-0015 (JP); SUHARA, Tetsuya, Chiba-shi Chiba 263-8555 (JP); HIGUCHI, Makoto, Chiba-shi Chiba 263-8555 (JP); HARADAHIRA, Terushi, Chiba-shi Chiba 263-8555 (JP); KI, Bin, Chiba-shi Chiba 263-8555 (JP)
(74) Representative: Grünecker, Kinkeldey, Stockmair & Schwanhäusser Anwaltssozietät
(86) International application number: PCT/JP2008/053385
(87) International publication number: WO 2008/108242

(57) **Abstract**

Brain-localizing polypeptides carrying a reactive group for linking to a molecule that does not have brain-localizing activity were successfully produced by introducing at least two lysine residues into cyclized polypeptides having a brain-localizing motif sequence. These polypeptides have improved metabolic stability compared to conventional brain-localizing polypeptides, and can efficiently translocate desired molecules into the brain.

## Description

### Technical Field

The present invention relates to brain-localizing cyclized polypeptides comprising a multivalent binding moiety, carrier molecules comprising these polypeptides, and test reagents that use these polypeptides.

### Background Art

Achieving an effective concentration of a drug or such by oral administration or injection is more difficult in the brain than in other organs because of the presence of the blood-brain barrier. While an effective drug concentration may be ensured by administering a large dose, this would mean infusing an excessive amount of the drug into peripheral blood, which would cause adverse effects such as kidney and liver damage. Therefore, it has become necessary to develop a system that selectively transports drugs to the brain. In that regard, numerous studies are being carried out. Most of the research and development involves efforts to enhance brain localization through chemical modification of the drug itself by utilizing a property of cerebrovascular endothelial cells - the higher the lipid solubility of a substance, the more easily it passes through the blood-brain barrier. Such methods improve drug localization in the brain by several folds at best, which is on the whole, no more than an error range. In the brain, contrary to peripheral organs where substances permeate through the intercellular spaces of vascular endothelial cells, the intercellular spaces of cerebrovascular endothelial cells form special structures called tight junctions and hardly allow permeation of blood components through them. Therefore, transport of substances to the brain must be carried out by permeation after the substances are chemically modified to be lipid-soluble and directly integratable into the cell membrane. More specifically, these methods make substances permeate directly into cells as there is no alternate route for substance transport to the brain, different from peripheral organs. However, since this mechanism is different from the usual, the efficiency is several thousands to tens of thousands times lower. Therefore, these methods cannot be referred to as brain-specific drug transport.

With recent technological advances, techniques that target membrane surface proteins expressed on cerebrovascular endothelial cells have been developed. In particular, it is effective to utilize the function of proteins called transporters for incorporating drugs into the brain. Since hardly any substances permeate into the brain through intercellular spaces as described above, amino acids and sugars in blood are specifically transported into the brain by binding to transporters expressed on the blood-brain barrier. Transferrin receptors are transporter molecules that transport proteins called transferrins to the brain. Transferrins supply metal ions to metalloenzymes which are necessary for brain activity. It was reported that using specialized antibodies to target transferrin receptors could increase the brain localization of drugs by several tens to approximately a hundred times (see Non-patent Document 1).

However, transferrin receptors are expressed not only in cerebrovascular endothelial cells, but also in liver and kidneys at an even larger quantity. Therefore, when this system is used, along with an increase in the amount of drug transported to the brain, the drug is also introduced into the liver and kidneys. Thus, this can hardly be called brain-specific transport. In addition, although there have been reports on systems that utilize several transporter molecules and antiporter molecules such as P-glycoproteins, none of them have been confirmed to be effective.

Furthermore, methods that utilize special functional peptides have been recently developed. These peptides are called PTD sequences, and were identified as peptide sequences necessary for HIV tat gene products to translocate into the cell nucleus. These peptides pass through not only the nuclear membrane, but all kinds of cell membranes (see Non-patent Document 2), and can therefore be distributed to organs throughout the entire body when injected into blood. PTD peptides can transport substances into the brain because they can pass through the cell membrane of cerebrovascular endothelial cells. However, although both PTD sequence-mediated transfer through the cell membrane and permeation from the intercellular space are effective in peripheral organs, the latter permeation is absent in the brain, making substance permeability much lower than in other organs. Therefore, this technique also cannot be brain-specific.

Meanwhile, molecules that regulate the organ specificity of vascular endothelial cells have been recently reported. Depending on the organ's role and specificity, each organ in the body has different nutritional requirements and different degrees of requirements for various factors supplied by blood. It is gradually becoming clear that vascular endothelial cells distributed in organs have slightly different characteristics depending on where they exist. Furthermore, vascular endothelial cells serve as direct contact points with inflammatory cells and immune cells present in blood, and control the invasion of these cells during inflammation and morbid conditions. Invading cells then accumulate at lesions by recognizing inflammatory homing receptors that appear during inflammation, as well as tissue-specific vascular endothelial cell marker molecules (called cellular zip codes). Although their roles are still unclear, these cell markers are attracting attention because targeting of these molecules can at least allow targeting of a molecule of interest up to vascular endothelial cells of an organ (Non-patent Document 3). However, although this method can target a molecule of interest up to vascular endothelial cells of each organ, systems for introducing the molecule into the parenchyma of an organ must be devised.

The present inventors obtained several polypeptides showing brain-localizing activity, and discovered from their polypeptide sequences, sequences of amino acid motifs that are involved in brain-localizing activity. Cyclized polypeptides comprising the motif sequences were confirmed to actually translocate into the brain tissues when administered to the body (see Patent Document 1).

Molecules that function inside the brain can be efficiently transported into the brain by linking to this brain-localizing polypeptide. For example, if the above-mentioned brain-localizing polypeptide can be linked to a PET ligand that does not have brain-localizing activity, thereby conferring the brain-localizing characteristic to the ligand, biological PET imaging of the brain may become possible.

For PET imaging, the ligand or peptide has to be labeled with a positron nuclide. Meanwhile, to link the ligand and peptide and to introduce a positron nuclide into the peptide, highly reactive substituents such as amino groups need to be present on the peptide. However, the problem is that because brain-localizing peptides have only one amino group available for reaction, it would not be possible to introduce positron nuclides into the peptides after they are attached to ligands.
(Patent Document 1) WO 2005/014625
(Non-patent Document 1) Ningya Shi and William M. Pardridge, Noninvasive gene targeting to the brain. Proc. Natl. Acad. Sci. USA, Vol. 97, Issue 13, 7567-7572, June 20, 2000
(Non-patent Document 2) Steven R. Schwarze, Alan Ho, Adamina Vocero-Akbani, and Steven F. Dowdy, In Vivo Protein Transduction: Delivery of a Biologically Active Protein into the Mouse. Science 1999 September 3; 285: 1569-1572.
(Non-patent Document 3) Renata Pasqualini, Erkki Ruoslahti, Organ targeting in vivo using phage display peptide libraries. Nature Vol.380, 28, March 1996.

### Disclosure of the Invention

### [Problems to be Solved by the Invention]

An objective of the present invention is to provide brain-localizing polypeptides comprising a reactive group for linking to molecules that do not have brain-localizing activity, and methods for efficiently linking brain-localizing polypeptides to molecules that do not have brain-localizing activity. Furthermore, an objective of the present invention is to provide brain-localizing carrier molecules that use these polypeptides and test reagents that use these polypeptides.

### [Means for Solving the Problems]

The present inventors conducted dedicated studies to solve the above-mentioned problems. As described above, linking a brain-localizing polypeptide sequence to a PET ligand that does not have brain-localizing activity confers the brain-localizing characteristic to the ligand, and may enable biological PET imaging of the brain. For PET imaging, the ligand or peptide has to be labeled with a positron nuclide, and to link the ligand and peptide and to introduce a positron nuclide into the peptide, highly reactive substituents such as amino groups need to be present on the peptide. However, brain-localizing polypeptides generally have only one amino group available for reaction, and a positron nuclide cannot be introduced into the peptide when a ligand had been attached. Thus, as it is, the ligand needs to be labeled in advance with a positron nuclide and then linked to the peptide. Since the lifetime of a positron nuclide is short (half-life: approximately 20 minutes for ¹¹C, and approximately 110 minutes for ¹⁸F), the ligand needs to be labeled and linked to the peptide in a short time; therefore, the types of positron-labeled ligands that can be linked to the peptide become limited, and setting the reaction conditions and purification conditions becomes difficult. Furthermore, brain-localizing polypeptides have cysteines (C) positioned at both ends of the brain-localizing motif and form a cyclic structure by using the SH group of C, but in this cyclic peptide, the S-S bond is unstable when the peptide is administered to the body of a living animal, and therefore the *in vivo* use is problematic. Consequently, the brain-localizing polypeptides are improved with an objective to develop a highly versatile positron nuclide-labeled brain-targeting peptide that is stable *in vivo* and enables PET imaging of molecules targeted by the ligand, by conferring the brain-localizing characteristic to a ligand that does not have brain-localizing activity.

Generally, as a method of introducing a positron nuclide into a polypeptide, the method of [¹⁸F] fluorobenzoyl ([¹⁸F]FB) -ation of a free amino group on the peptide is common. This method is easily accomplished by reacting a peptide carrying an amino group with an activated ester of [¹⁸F]FB ([¹⁸F]SFB), and allows introduction of the ¹⁸F atom which has a relatively long lifespan (half-life of approximately 110 minutes) as a positron nuclide. Furthermore, if there is another free amino group present on this peptide, a ligand can be attached to that amino group. Consequently, the cysteines (C) at the terminal regions of the brain-localizing polypeptide were substituted with lysines (K) carrying an ε-amino group to synthesize a peptide comprising two amino groups. For the cyclization of this lysine (K)-substituted peptide, the amide bond formation between N- and C-termini is considered to be suitable as a method for efficient and low-cost production without affecting the fundamental side-chain structure necessary for brain-localizing activity. Thus, by forming an amide bond between the N- and C-termini of the lysine (K)-substituted peptide, a cyclized polypeptide that maintains the cyclic structure essential for brain-localizing activity was prepared.

As a result, polypeptides cyclized with an amide bond formed between lysines at the ends were more stable in *in vivo* metabolism than conventional polypeptides cyclized through cysteines. That is, enhancement of metabolic stability *in vivo* was observed for the first time by introducing a lysine amide bond into a cyclized polypeptide.

To verify the translocation of the cyclized polypeptide of the present invention into the cerebral parenchyma, PET imaging was performed on the cyclized polypeptide of the present invention labeled with a positron nuclide. As a result, the cyclized polypeptide of the present invention was confirmed to rapidly translocate into the cerebral parenchyma after administration to the carotid artery.

Next, the inventors examined to see whether the cyclized polypeptide of the present invention can transport another molecule into the brain. A PET ligand was used as the molecule to be transported into the brain, and this PET ligand was attached to the cyclized polypeptide of the present invention labeled with a positron nuclide. Results of the PET imaging analysis showed that the cyclized polypeptide can translocate the ligand to the cerebral parenchyma through administration into the carotid artery.

The present inventors successfully introduced reactive groups (bonding moiety) into the brain-localizing polypeptide for linking to other molecules by cyclizing the brain-localizing polypeptide through an amide bond between two lysines. It was confirmed that by linking to a molecule, the brain-localizing polypeptide of the present invention can translocate the molecule efficiently into the brain. Furthermore, surprisingly the lysine-containing cyclized polypeptide of the present invention was found to achieve an effect of dramatically enhancing resistance to degradation *in vivo*.

The present invention relates to brain-localizing polypeptides comprising reactive groups to be used for linking to molecules that do not have brain-localizing activity, and methods for efficiently linking brain-localizing polypeptides to molecules without brain-localizing activity, carrier molecules that use these polypeptides for localization into the brain, and test reagents that use these polypeptides. More specifically, the present invention provides:
[1] a cyclized polypeptide comprising a multivalent binding moiety, which is a cyclized polypeptide comprising a brain-localizing motif sequence and at least two lysine residues;
[2] the cyclized polypeptide of [1], comprising two adjacent lysine residues;
[3] the cyclized polypeptide of [1] or [2], wherein metabolic stability is improved;
[4] the cyclized polypeptide of any one of [1] to [3], wherein a PET nuclide-labeled molecule is attached to the ε-amino group of lysine, and/or a ligand molecule of a brain receptor is attached to the ε-amino group of another lysine;
[5] a carrier molecule for brain localization comprising the cyclized polypeptide of any one of [1] to [3] as an active ingredient;
[6] a brain-localizing pharmaceutical agent, wherein a pharmaceutical agent is attached to the ε-amino group of a lysine residue of the cyclized polypeptide of any one of [1] to [3];
[7] a reagent for PET examination, which comprises the cyclized polypeptide of [4] as an active ingredient;
[8] a kit for producing a brain-localizing pharmaceutical agent, comprising at least the following substances as components:
   (a) the cyclized polypeptide of any one of [1] to [3]; and
   (b) a pharmaceutical agent;
[9] a kit for PET examination, which comprises at least the following substances as components:
   (a) a cyclized polypeptide of any one of [1] to [3]; and
   (b) a molecule labeled with a PET nuclide;
[10] a method for producing a cyclized brain-localizing polypeptide comprising a multivalent binding moiety, which comprises the step of forming an amide bond between the two ends of a polypeptide comprising lysines at both ends and a brain-localizing motif sequence;
[11] a method for producing a brain-localizing polypeptide with improved metabolic stability, which comprises the step of forming an amide bond between the two ends of a polypeptide comprising lysines at both ends and a brain-localizing motif sequence;
[12] a method for producing a brain-localizing pharmaceutical agent, comprising the step of attaching the ε-amino group of a lysine of the cyclized polypeptide of any one of [1] to [3] to a pharmaceutical agent;
[13] a method for producing a regent for PET examination, comprising the step of attaching the ε-amino group of a lysine of the cyclized polypeptide of any one of [1] to [3] to a molecule labeled with a PET nuclide;
[14] a method for producing a brain-localizing ligand molecule, comprising the step of attaching the ε-amino group of a lysine of the cyclized polypeptide of any one of [1] to [3] to a ligand molecule for a brain receptor;
[15] a method for cyclizing a brain-localizing polypeptide in a state that carries a multivalent binding moiety, comprising the step of forming an amide bond between the two ends of a polypeptide comprising lysines at both ends and a brain-localizing motif sequence; and
[16] a method of improving metabolic stability of a brain-localizing polypeptide, comprising the step of forming an amide bond between the two ends of a polypeptide comprising lysines at both ends and a brain-localizing motif sequence.
   The present invention also provides the following:
[17] use of a polypeptide comprising lysines at both ends and a brain-localizing motif sequence in a method for cyclizing a brain-localizing polypeptide with a multivalent binding moiety; and
[18] use of a polypeptide comprising lysines at both ends and a brain-localizing motif sequence in a method for improving the metabolic stability of a brain-localizing polypeptide.

### Brief Description of the Drawings

Fig. 1 shows schematic diagrams of [C]K004K, [C]C004C, and [C]C004CY.
Fig. 2 shows a comparison of *in vitro* metabolism of [C]C004CY[¹³¹I]. Blood: 1/5 homogenate 400 µL; Brain: 1/5 homogenate 400 µL; Liver: 1/5 homogenate 400 µL; Plasma: x1 50 µL.
Fig. 3 shows the results of *in vivo* metabolism test by TLC analysis.
Fig. 4 shows a schematic diagram of [C]([¹⁸F]FB)-K004K-(L-703,717) synthesis.
Fig. 5 shows a diagram and a graph showing the results of peptide metabolism test in a liver extract solution. (A) is a diagram showing the brain-targeting peptide sequence, and (B) is a graph showing the change over time in the proportion of unchanged peptide in the liver extract solution.
Fig. 6 shows imaging photographs obtained when ([¹⁸F]FB)-K004K is administered to the right common carotid artery of a mouse. (A-C) PET imaging, (D) *ex vivo* ARG.
Fig. 7 shows imaging photographs obtained when ([¹⁸F]FB)-K004K is administered to the mouse tail vein.
Fig. 8 shows imaging photographs of ([¹⁸F]FB)-K004K-(L-703,717). (A-C) PET imaging, (D) *ex vivo* ARG.
Fig. 9 shows the results of *ex vivo* ARG of the rat carotid artery administration.
Fig. 10 shows a diagram, tables, and photographs relating to the PSL curve and brain-localizing ratio determined by ARG.

### Best Mode for Carrying Out the Invention

The present invention provides cyclized polypeptides comprising a brain-localizing motif sequence, which comprise a multivalent binding moiety. Such polypeptides have the characteristic of containing at least two lysine residues (hereinafter, they may be described as "polypeptides of the present invention"). Polypeptides of the present invention have the characteristic of improved metabolic stability.

In general, translocation of substances from blood into brain tissue is restricted by a structure called the blood-brain barrier (BBB). This structure protects the brain from harmful substances. In the present invention, "brain-localizing activity" or "brain-localizing characteristic" refers to the activity of molecules, such as polypeptides administered to the body (for example, by intravenous administration), to translocate into brain tissues. The polypeptides of the present invention can ordinary be described as polypeptides having brain-localizing activity (brain-localizing polypeptides), but they can also be, for example, described as polypeptides having the ability to pass through the blood-brain barrier or to induce transmigration (transcytosis). The polypeptides of the present invention can be attached to other substances (molecules) to translocate them into the brain. Therefore, the polypeptides of the present invention can be referred to as "polypeptides that confer brain-localizing activity", "brain-localizing peptide tags", or "agents that confer brain-localizing activity".

Brain-localizing polypeptides in the present invention are cyclic, and while the number of amino acids constituting these polypeptides is not particularly limited, it is for example 100 amino acids or less, preferably 15 amino acids or less, and more preferably 9 amino acids or less, and most preferably 4 to 9 amino acids.

In the present invention, a brain-localizing motif is, for example, the amino acid motif sequence of [Sequence 1], more preferably the amino acid motif sequence of [Sequence 2], or the amino acid motif sequence of [Sequence 3] below (WO 2005/014625).
[Sequence 1] X₁-(R or K)-X₃-X₄ or
   X₄-X₃-(R or K)-X₁,
   wherein
   X₁ denotes S (serine), T (threonine), N (asparagine), P (proline), V (valine), or L (leucine);
   X₃ denotes an arbitrary amino acid; and
   X₄ denotes G (glycine), S (serine), T (threonine), C (cysteine), N (asparagine), L (leucine), Q (glutamine), or Y (tyrosine).
[Sequence 2] X₁-(R or K)-X₃-X₄ or
   X₄-X₃-(R or K)-X₁,
   wherein
   X₁ denotes S (serine), T (threonine), N (asparagine), P (proline), or V (valine), and is preferably S or T;
   X₃ denotes an arbitrary amino acid; and
   X₄ denotes G (glycine), S (serine), T (threonine), C (cysteine), N (asparagine), Q (glutamine), or Y (tyrosine), and is more preferably T, Q, or C. In the above-mentioned (R or K), R is more preferable.

These amino acids (G, S, T, C, N, Q, and Y) are generally categorized into uncharged polar amino acids.
[Sequence 3] X₁-(R or K)-X₃-X₄ or
   X₄-X₃-(R or K)-X₁,
   wherein
   X₁ denotes S (serine), T (threonine), P (proline), or L (leucine);
   X₃ denotes an arbitrary amino acid; and
   X₄ denotes G (glycine), S (serine), T (threonine), C (cysteine), L (leucine), or Q (glutamine).

Herein, the amino acids are described using the conventional single letter code (for example, R for arginine and K for lysine). Furthermore, the amino acid sequences are written in the order from N terminus to C terminus according to conventional description methods.

Polypeptides of the present invention are cyclized polypeptides comprising the above-mentioned motif sequence and at least two lysine residues.

The number of lysine residues included in the cyclized polypeptide is not particularly limited so long as it is two or more, but generally, there are preferably two or more lysine residues excluding the ones present in the motif sequence. The positions where the lysines exist are not particularly limited as long as they are outside the region constituting the motif sequence, the lysines may be adjacent to each other (-KK-), or the two lysines may be positioned so that one or more other amino acids are included between them (-KXK-; herein, X represents any amino acid, and the number of X's is not limited), but preferably the two lysine residues are adjacent to each other. More specifically, the lysine residues preferably form an amide bond with each other.

In the present invention, "binding moiety" refers to a reactive group for linking a polypeptide of the present invention to another molecule (for example, a molecule comprising a peptide). Specifically, it refers to one of the two amino groups in lysine, and more specifically refers to the ε-amino group of lysine.

The number of binding moieties carried by a polypeptide of the present invention usually increases according to the number of lysine residues. For example, when it contains two lysines, there are generally two binding moieties, and such a case is called a bivalent binding moiety in the present invention. When it contains multiple binding moieties, this is referred to as a polyvalent (multivalent) binding moiety in the present invention.

Polypeptides of the present invention are polypeptides comprising a cyclic structure. In the present invention, the above-mentioned motif sequence can be found in the polypeptides constituting this cyclic region. The amino acids constituting the motif sequence consists of four amino acid residues adjacent to each other. These adjacent amino acids usually form peptide bonds (amide bonds) with each other.

Herein, the symbol "-" in the above-mentioned [Sequence 1] to [Sequence 3] usually means a peptide bond.

Furthermore, in a preferred embodiment of the present invention, as long as a polypeptide comprises an above-mentioned motif sequence consisted of four amino acids and including two or more lysine residues, the non-motif amino acid sequence of the polypeptide is not particularly limited.

The polypeptides having brain-localizing activity described in Table 1 have the following characteristics.

In the polypeptide regions that may form a cyclic structure (more specifically, the amino acid sequences excluding the cysteines at both ends), (1) all polypeptides comprise a basic amino acid, K or R, and (2) the remaining amino acid residues consist of any of the 10 amino acids [G, A, V, L, S, T, P, Q, H, and N].

Therefore, a preferred embodiment of the present invention provides polypeptides having brain-localizing activity, polypeptides comprising a cyclic region in which at least one or more basic amino acid residues (K or R) are present, and the remaining amino acid residues (usually 80% or more, preferably 85% or more, more preferably 90% or more, even more preferably 95% or more, and most preferably 100%) are selected from the group of amino acid residues [G, A, V, L, S, T, P, Q, H, and N] (this characteristic may be referred to herein as "Feature 1 "). In a more preferable embodiment of the present invention, polypeptides have the above-mentioned "Feature 1" and comprise a motif sequence ([Sequence 1] to [Sequence 3]) of the present invention.

Therefore, in the present invention, polypeptides comprising brain-localizing motif provides, for example, the following polypeptides:
(a) a polypeptide having brain-localizing activity, in which the polypeptide comprises 10% or more basic amino acid residues (K or R);
(b) a polypeptide having brain-localizing activity, in which the polypeptide comprises a cyclic peptide region and 10% or more basic amino acid residues (K or R) in the cyclic peptide region; and
(c) a polypeptide having brain-localizing activity, wherein the polypeptide comprises a cyclic peptide region and at least one or more basic amino acid residues (K or R) in the cyclic peptide region.

In a preferred embodiment of the present invention, polypeptides are cyclized polypeptides comprising above-mentioned polypeptides and having two or more lysine residues.

In a preferred embodiment of the present invention, the length of a polypeptide region to be cyclized is, not particularly limited, for example, 100 amino acids or less, preferably 50 amino acids or less, more preferably 4 to 30 amino acids, even more preferably 4 to 15 amino acids, yet even more preferably 4 to 9 amino acids, and most preferably 4 to 7 amino acids.

Furthermore, in a preferred embodiment, the polypeptides of the present invention comprise function (activity) (A) and/or (B) below:
(A) transmigration (transcytosis)-inducing activity
(B) cerebrovascular endothelial cell-binding activity.

The term "transmigration" in (A) refers to a phenomenon in which certain molecules penetrate into the brain by passing through vascular endothelial cells rather than intercellular spaces of the vascular endothelial cells. This is called "trans-endothelial cell migration", "transcellular pathway", or "transcytosis". The molecules (cells and such) that pass through vascular endothelial cells by this mechanism may have signal molecules on their surface and induce the above-mentioned phenomenon in the vascular endothelial cells through receptors on the surface of these cells.

The polypeptides of the present invention may have an activity to induce transmigration in vascular endothelial cells. More specifically, the polypeptides of the present invention may serve as signal molecules for inducing transmigration.

Signal molecules are thought to bind to cerebrovascular endothelial cells (for example, receptors on the cells) in the early stages of transmigration. Therefore, in a preferred embodiment, one of the characteristics of the polypeptides of the present invention is to have an activity to bind to cerebrovascular endothelial cells.

Whether an arbitrary test molecule has the transmigration-inducing activity of (A) or whether it has the cerebrovascular endothelial cell-binding activity of (B) can be evaluated appropriately using methods known to those skilled in the art. As an example, the evaluation can be carried out by administering a fluorescence-labeled test molecule into blood vessels, and then observing frozen cross-sections of cerebrovascular endothelial cells under a fluorescence microscope. For example, if vascular endothelial cells to which a fluorescence-labeled test molecule is attached are observed, the test molecules are judged to have the activity of (B), and if the fluorescence-labeled test molecules are detected within the vascular endothelial cells, the test molecules are judged to have the activity of (A).

In addition to the fluorescence-labeling method, also included are methods using isotope labels or PET ligand labels, detection methods using MRI after binding with a magnetite or the like, etc. Besides the above-mentioned *in vivo* methods, other embodiments include methods of evaluating transmigration-inducing activity by establishing a blood-brain barrier (BBB) model using vascular endothelial cell culture, and then administering the above-mentioned test molecule. If the molecule is confirmed to permeate through the BBB, it is judged to have the activity of (A), and if the molecule is adhered to the vascular endothelial cells after washing, it is judged to have the activity of (B).

Furthermore, the phrase "cerebrovascular endothelial cells" in the present invention can refer to cells such as mouse MBEC4, commercially available human cerebrovascular endothelial cell BBEC, temporary cultured bovine cerebrovascular endothelial cells, or co-cultures of peripheral blood vessel-derived vascular endothelial cells and astrocytes prepared for inducing a BBB-like function.

Those skilled in the art can use these cells to evaluate the activity of (A) or (B) in arbitrary polypeptides (synthetic peptides), or molecules comprising these peptides.

Specifically, polypeptides comprising a sequence produced by removing the cysteine residues at both ends in the sequences below can be shown favorably as polypeptides comprising a motif sequence in the present invention.

**Table 1**

| Name | Amino acid sequence |
|---|---|
| T2J001 | CSNLLSRHC (SEQ ID NO: 1) |
| T2J002 | CSLNTRSQC (SEQ ID NO: 2) |
| T2J003 | CVAPSRATC (SEQ ID NO: 3) |
| T2J004 | CVVRHLQQC (SEQ ID NO: 4) |
| T2J004V3L | CVLRHLQQC (SEQ ID NO: 5) |
| T2J006 | CRQLVQVHC (SEQ ID NO: 6) |
| T2J007 | CGPLKTSAC (SEQ ID NO: 7) |
| T2J008 | CLKPGPKHC (SEQ ID NO: 8) |
| T2J009 | CRSPQPAVC (SEQ ID NO: 9) |
| T2J012 | CNPLSPRSC (SEQ ID NO: 10) |
| T2J013 | CPAGAVKSC (SEQ ID NO: 11) |
| T2J013V6L | CPAGALKSC (SEQ ID NO: 12) |

Therefore, in the present invention, a brain-localizing motif sequence is, for example, a sequence of any one of SEQ ID NOs: 13 to 24.

A preferred embodiment of the present invention is, for example, a cyclized polypeptide having a structure in which cysteine residues at both ends of the above-mentioned polypeptide are substituted to lysines which are then linked by an amide bond.

In a preferred embodiment of the present invention, "a cyclized polypeptide comprising a brain-localizing motif sequence" is, for example, a cyclized polypeptide which has an amino acid sequence comprising the brain-localizing motif sequence of any one of SEQ ID NOs: 13 to 24, and has a structure in which lysine is added to both ends of the sequence, and the lysines are linked to each other through an amide bond (its chain length is for example 100 amino acids or less, preferably 50 amino acids or less, more preferably 30 amino acids or less, and even more preferably 15 amino acids or less, and yet even more preferably 12 to 9 amino acids, and most preferably 9 amino acids).

More preferably, "a cyclized polypeptide comprising a brain-localizing motif sequence" is, for example, a cyclized polypeptide having a structure in which lysine is added to both ends of the brain-localizing motif sequence of any one of SEQ ID NOs: 13 to 24, and the lysines are linked to each other through an amide bond.

Furthermore, the present invention includes polypeptides produced by suitably modifying a cyclized polypeptide having a structure in which the cysteine residues at the two ends of a polypeptide of any one of the above-mentioned SEQ ID NOs: 1 to 12 have been substituted to lysines which are then linked through an amide bond.

That is, polypeptides comprising an amino acid sequence with one or multiple amino acid additions, deletions, or substitutions in the amino acid sequence of a cyclized polypeptide, which are polypeptides functionally equivalent to a polypeptide of the present invention are included in the present invention. The phrase "functionally equivalent" refers to, for example, brain-localizing activity. The above-mentioned term "multiple" is not particularly limited so long as it is two or more, but it is generally a small number of approximately two to nine, preferably two to five, and more preferably two or three.

When specific amino acid sequences (for example, SEQ ID NOs: 1 to 12) are disclosed, one skilled in the art can produce cyclized polypeptides comprising a sequence with suitable amino acid modifications based on these amino acid sequences, and can suitably select polypeptides of the present invention by evaluating whether or not the produced polypeptides have brain-localizing activity. The method of evaluating for the presence or absence of brain-localizing activity in desired polypeptides can be carried out as described later in the Examples, for example, by administering a test polypeptide to the carotid artery of an animal and evaluating whether or not it translocates into the brain of that animal.

Organisms in which the polypeptides of this invention show brain-localizing activity are not particularly limited as long as they are animals that have a blood-brain barrier, but are usually mammals, and preferably mice, rats, gerbils, cats, cattle, monkeys, or humans.

The polypeptides of the present invention may be polypeptides derived from natural proteins, polypeptides derived from recombinant proteins, chemically synthesized polypeptides, or such. Those skilled in the art can synthesize polypeptides comprising any amino acid sequence, and cyclize those peptides.

For example, synthesis of a polypeptide having the above-mentioned motif sequence can be carried out suitably by methods known to those skilled in the art, for example, using a commercially available polypeptide synthesizer or such.

Furthermore, the present invention also includes straight-chain polypeptides having a structure in which any amide bond in a cyclized polypeptide of the present invention is cleaved. Those skilled in the art can easily cyclize the straight-chain polypeptide of interest by linking its ends to each other.

The cyclized polypeptides of the present invention can be produced, for example, by cyclizing the above-mentioned straight-chain polypeptides. The above-mentioned straight-chain polypeptides can be produced, for example, by expressing vectors inserted with polynucleotides encoding the polypeptides in host cells.

Such vectors are not particularly limited as long as the inserted DNA is stably maintained. For example, when using *E. coli* as host, the cloning vector is preferably a pBluescript vector (Stratagene) and such. Expression vectors are particularly useful as vectors for producing the polypeptides of the present invention. Expression vectors are not particularly limited as long as they can express polypeptides in test tubes, *E. coli*, cultured cells, or individual organisms. For example, preferred vectors are pBEST vector (Promega) for expression in test tubes, pET vector (Invitrogen) for *E. coli*, pME18S-FL3 vector (GenBank Accession No. AB009864) for cultured cells, pME18S vector (Mol. Cell Biol. (1988) 8: 466-472) for individual organisms. Insertion of a DNA of the present invention into vectors can be performed by standard methods such as ligase reactions using restriction enzyme sites (Current protocols in Molecular Biology edit. Ausubel et al. (1987) Publish. John Wiley & Sons. Section 11.4-11.11).

The host cells into which the vector is introduced are not particularly limited, and various host cells can be used depending on the purpose. Cells used for expressing the polypeptides include bacterial cells (for example, *Streptococcus, Staphylococcus, E. coli, Streptomyces,* and *Bacillus subtilis*), fungal cells (for example, yeast and *Aspergillus*), insect cells (for example, *Drosophila* S2 and *Spodoptera* SF9), animal cells (for example, CHO, COS, HeLa, C127, 3T3, BHK, HEK293, and Bowes melanoma cell), and plant cells. Vectors can be introduced into host cells using known methods such as the calcium phosphate precipitation method, electroporation method (Current protocols in Molecular Biology edit. Ausubel et al. (1987) Publish. John Wiley & Sons. Section 9.1-9.9), lipofectamine method (GIBCO-BRL), and microinjection method.

For secreting host cell-expressed polypeptides into the lumen of endoplasmic reticulum, periplasmic space, or extracellular environment, suitable secretion signals can be incorporated into the polypeptides of interest. These signals may be intrinsic or foreign to the polypeptides of interest.

When the polypeptides are secreted into culture media, they are collected by harvesting the media. When the polypeptides are produced inside cells, the cells are lysed to collect these polypeptides.

These polypeptides can be collected and purified from recombinant cell cultures by known methods including ammonium sulfate or ethanol precipitation, acidic extraction, anion or cation exchange chromatography, phosphocellulose chromatography, hydrophobic interaction chromatography, affinity chromatography, hydroxyapatite chromatography, and lectin chromatography.

A polypeptide of the present invention can be produced by forming an amide bond between the two ends of a polypeptide that comprises a brain-localizing motif sequence and has lysines at both ends. As preferred embodiments of the present invention, methods are provided for producing a cyclized brain-localizing polypeptide having a multivalent binding moiety, which comprise the step of forming an amide bond between the two ends of a polypeptide that comprises a brain-localizing motif sequence and has lysines at both ends.

The above-mentioned phrase "has lysines at the ends" is not necessarily limited to cases in which the lysine is positioned at the terminus, and includes cases in which it is positioned at position two or further from the terminus.

Polypeptides of the present invention produced by the above-mentioned production methods have the characteristic of being cyclized in a state that carries a multivalent binding moiety.

Therefore, the present invention relates to methods for cyclizing a brain-localizing polypeptide in a state that carries a multivalent binding moiety, which comprises the step of forming an amide bond between both ends of a polypeptide that comprises a brain-localizing motif sequence and has lysines at both ends.

Furthermore, polypeptides of the present invention show exceptional metabolic stability when administered into the living body of an animal. Therefore, the present invention provides methods for producing a brain-localizing polypeptide with improved metabolic stability, which comprise the step of forming an amide bond between the two ends of a polypeptide that comprises a brain-localizing motif sequence and has lysines at both ends. The present invention also relates to methods for improving the metabolic stability of brain-localizing polypeptides, which comprise the step of forming an amide bond between both ends of a polypeptide that comprises a brain-localizing motif sequence and has lysines at both ends.

The present invention also provides methods for translocating an arbitrary molecule to the brain of an animal. These methods are preferably methods comprising steps (a) and (b) below:
(a) producing a brain-localizing cyclized polypeptide having a structure in which an arbitrary molecule and a polypeptide of the present invention are attached; and
(b) administering the above-mentioned cyclized polypeptide into the body of an animal.

Since the polypeptides of the present invention have brain-localizing activity, brain-localization activity is conferred to molecules attached to the polypeptides of the present invention. Therefore, polypeptides of the present invention are thought to have the function of conferring brain-localizing activity to an arbitrary molecule by forming a bond with the arbitrary molecule. That is, polypeptides of the present invention have useful applications which may confer brain-localizing activity to other molecules. The present invention provides pharmaceutical agents for conferring brain-localizing activity to an arbitrary molecule, which comprise polypeptides of the present invention. In a preferred embodiment, the present invention provides peptide tags for translocating an arbitrary molecule to the brain, which comprises a polypeptide having the above-mentioned amino acid motif sequence.

Examples of the peptide tags of the present invention include conjugate molecules formed between the peptides of the present invention and biotin. Such conjugate molecules can be linked appropriately with any avidin-conjugated molecule. By using these conjugate molecules, desired avidin-conjugated molecules can be translocated into the brain. Methods of translocating any molecule into the brain by using these conjugate molecules are also encompassed in the present invention. Furthermore, the present invention comprises molecules that have been conferred with a brain-localizing activity by a pharmaceutical agent of the present invention mentioned above.

Molecules that are conferred with a brain-localizing activity by the polypeptides (pharmaceutical agents) of the present invention are not particularly limited, and include: single compounds such as natural compounds, organic compounds, inorganic compounds, carbohydrate chains, proteins, and peptides; as well as compound libraries, expression products of gene libraries, cells, cell extracts, cell culture supernatants, microorganisms, products of microorganisms, phages, antigens, antibodies, micelles (polymeric micelles and such), liposomes, microcapsules, peptide nucleic acids (PNAs), and pharmaceutical compounds. The polypeptides of the present invention or the aforementioned molecules can be appropriately labeled if necessary. The labels include radioactive labels, fluorescence labels, and enzyme labels.

The size of the molecules that are conferred with a brain-localizing activity by the polypeptides of the present invention is not particularly limited, but the maximum size is generally a size that allows the molecules to physically pass through the blood-brain barrier. Molecules with the size of a standard phage can pass through the blood-brain barrier *via* the action of the polypeptides of the present invention. Therefore, these molecules (substances) may have sizes similar to that of a phage. For example, if the molecules to be conferred with brain-localizing activity are made up of amino acids, they may comprise around 100,000 amino acids.

Those skilled in the art can use known methods to suitably bind an above-mentioned molecule to a polypeptide of the present invention according to the type of the molecule.

For example, the molecules and polypeptides of the present invention can be linked by methods that use commercially available coupling reagents (N-binding type, COOH-binding type, amino acid residue modifying type, S-S linkage type, and so on), methods using chloramine T, methods that introduce isothiocyanate groups, and such.

Furthermore, when the molecule is a protein, the molecule to which a polypeptide of the present invention is linked can be prepared by using DNAs that encode a fusion protein between the protein and the polypeptide of the present invention.

The present invention also provides carrier molecules for delivery to the brain, wherein the carriers comprise a cyclized polypeptide of the present invention having brain-localizing activity as an active ingredient. These carriers may also be referred to as "supports" or "transporters". By directly linking a polypeptide of the present invention to a molecule to be translocated to the brain, the molecule can be translocated to the brain. Furthermore, a preferred embodiment of the carriers of the present invention includes carriers comprising a structure in which a polypeptide of the present invention is bound to a micelle (polymeric micelle), liposome, or microcapsule.

Furthermore, by using the carriers of the present invention, desired pharmaceutical agents can be translocated to the brain. For example, by using a carrier to support a compound (pharmaceutical composition) that has a therapeutic effect on a brain disease, the compound can be translocated efficiently to the brain and exert powerful therapeutic effects. Carriers used to support a compound (pharmaceutical composition) are themselves expected to be therapeutic agents for brain diseases. Accordingly, the present invention provides therapeutic agents for brain diseases, comprising a structure in which a drug is supported on a present invention's carrier for translocation to the brain. "Supported" may refer to conditions in which a drug is directly bound to a carrier, or conditions in which a drug (pharmaceutical composition) is contained within a carrier.

In addition, the present invention provides methods for producing molecules having brain-localizing activity of the present invention. In a preferred embodiment of the present invention, the method for producing molecules having brain-localizing activity comprises binding a polypeptide of the present invention to an arbitrary molecule.

Moreover, it is preferable that the polypeptides of the present invention for binding to molecules to be conferred with brain-localizing activity are positioned on the outside of these molecules. More specifically, it is desirable that the polypeptides of the present invention are bound to the molecules in such a way that the polypeptides are positioned on the surface of the molecules.

Examples of preferred embodiments of the present invention include polypeptides of the present invention comprising a sequence that positions the above-mentioned motif sequence between the lysine residues (K). Formation of an amide bond (peptide bond) between the N-terminal α-amino group and the C-terminal carboxyl group of such a polypeptide is expected to make a polypeptide chain containing the motif sequence positioned in between to protrude in the form of a loop.

Examples of molecules to be bound to the polypeptides of the present invention include compounds that are desirable for direct translocation into brain tissues for brain disease treatment. These compounds are conferred with brain-localizing activity by the polypeptides of the present invention. Consequently, when these compounds are administered into a body, they are expected to translocate efficiently into brain tissues and exert therapeutic effects. In this case, in addition to cranial nerve diseases, brain diseases include spinal nerve diseases or diseases of the myelin sheath such as multiple sclerosis, as well as various types of brain tumors or metastatic brain tumors coming from tumors originating in other organs.

Methods for producing brain-localizing pharmaceutical agents, comprising the step of attaching the ε-amino group of a lysine of a polypeptide of the present invention to a pharmaceutical agent, are also included in the present invention.

Furthermore, in a preferred embodiment of the present invention, a polypeptide of the present invention is, for example, used in PET imaging (PET examination and such).

For example, a molecule labeled with a PET nuclide can be attached to the ε-amino group of a lysine included in a polypeptide of the present invention. The manner in which a PET-nuclide-labeled polypeptide of the present invention translocates to the brain can be observed by biological imaging analysis of the brain.

For the above-mentioned "PET nuclide", a positron nuclide used for conventional PET examination can be used suitably. Specific examples include ¹¹C (carbon-11), ¹³N (nitrogen-13), ¹⁵O (oxygen-15), ¹⁸F (fluorine-18), ⁶²Cu (copper-62), ⁶⁸Ga (gallium-68), and ⁸²Rb (rubidium-82).

For example, by linking a polypeptide of the present invention to a ligand for a molecule that plays an important role in biological functions such as a brain receptor, one can examine functions of the biologically functional molecule, and screen for substances that inhibit the binding between the ligand and biologically functional molecule. Specifically, examples of ligands for brain receptors include the L-703,717 succinimide derivative which is a ligand for the *N*-methyl-D-aspartate receptor (a type of cranial nerve receptor), and ¹¹C-MQNB (¹¹C-labeled methylquinuclidinyl benzilate) which does not have brain-localizing characteristic and is an imaging ligand for the cardiac muscarinic receptor.

In a preferred embodiment of the present invention, the present invention provides cyclized polypeptides in which a molecule labeled with a PET nuclide is attached to the ε-amino group of a lysine, and a ligand molecule for a brain receptor is attached to the ε-amino group of another lysine. These polypeptides are useful as reagents for PET examination.

The present invention also includes methods for producing a reagent for PET examination, comprising the step of attaching the ε-amino group of a lysine of the polypeptide of the present invention to a molecule labeled with a PET nuclide.

When necessary, the above-mentioned production method includes the step of linking a PET ligand molecule or such to the polypeptide of the present invention.

Furthermore, the present invention also includes methods for producing brain-localizing ligand molecules, comprising the step of attaching the ε-amino group of a lysine of the polypeptide to a ligand molecule for a brain receptor.

The present invention includes molecules having brain-localizing activity, which are expected to have therapeutic effects against brain diseases such as those described above, and pharmaceutical agents containing such molecules.

A pharmaceutical agent of the present invention may comprise a polypeptide of the present invention, or a molecule that comprises the polypeptide and has brain-localizing activity; or may be formulated using a known pharmaceutical preparation method. For example, the agent can be formulated into a pharmaceutical formulation suitable for effective administration into the body, such as an injection (preferred), transnasal formulation, transdermal formulation, or oral agent, by suitably combining with an appropriate conventionally used carrier or vehicle, such as sterilized water, physiological saline, vegetable oil (for example, sesame oil and olive oil), coloring agent, emulsifier (for example, cholesterol), suspending agent (for example, gum arabic), surfactant (for example, polyoxyethylene hardened castor oil surfactants), solubilizing agent (for example, sodium phosphate), stabilizer (for example, sugars, sugar alcohols, and albumin), or preservative (for example, paraben). For example, injection formulations can be provided as freeze-dried products, solutions for injections, or such.

Furthermore, administration into the body can be carried out, for example by intraarterial injection, intravenous injection, or subcutaneous injection, and also intranasally, transbronchially, intramuscularly, or orally by methods known to those skilled in the art. Among these, intraarterial administration is preferred.

The present invention also provides kits for producing a brain-localizing pharmaceutical agent, comprising at least a polypeptide of the present invention and a pharmaceutical agent showing pharmacological activity in the brain as components.

Furthermore, the present invention provides a kit for PET examination, which comprises at least a polypeptide of the present invention and a molecule labeled with a PET nuclide as components. When necessary, the kit may further include a ligand molecule for a brain receptor.

In addition to the above-described components, the above-mentioned kits may be produced by combining reaction solutions, buffers, cells, polynucleotides, antibodies, various types of reagents to be used in a detector, model animals, or such that may be used in the methods of the present invention. Furthermore, instructions or such describing the method for using the kits can be packaged in the kits.

All prior art references cited herein are incorporated by reference.

### Examples

Herein below, the present invention will be specifically described using the Examples; however, it is not to be construed as being limited thereto.

### [Experiment Technique]

### Synthesis ofN-succinimidyl-4-[18F]fluorobenzoate ([18F]SFB)

*t*-Butyl 4-*N,N,N*-trimethylammoniumbenzoate (5 mg) was used as a starting material, reacted with [¹⁸F]KF/Kryptofix 222 in acetonitrile (0.5 mL) (90°C for 10 minutes), and subsequently hydrolyzed with hydrochloric acid (100°C for 5 minutes), and the obtained 4-[¹⁸F] fluorobenzoic acid was purified using C-18 Sep-Pak. To the purified intermediate, 4-[¹⁸F] fluorobenzoic acid, *O*-(*N*-succinimidyl)-*N,N,N',N'*-tetramethyluronium tetrafluoroborate (TSTU, 15 mg) was added in acetonitrile (1 mL) in the presence of 25% tetramethylammonium hydroxide (20 µL), heated (90°C for 5 minutes), and after cooling, the reaction solution was collected in a 5% acetic acid solution (10 mL). [¹⁸F]SFB was purified using C18 Sep-Pak, by washing with 10% acetic acid, followed by elution with 3 mL of dichloromethane. The eluate was dried under a stream of nitrogen.

The process of [¹⁸F]SFB synthesis is shown below.

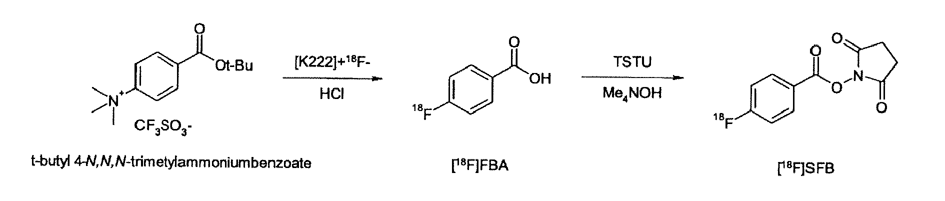

### Synthesis of the L-703,717 succinimide derivative

To an N,N-dimethylformamide (DMF) solution (1 mL) containing FE-21 (22.8 mg, 60 µmol), 6-bromohexanoic acid (11.77 mg, 60 µmol) was added in the presence of 60% NaH (12.07 mg, 303 µmol), and this was heated for two hours at 60°C. After addition of deionized water and 0.05 N HCl, the reaction mixture was extracted using ethyl acetate. The ethyl acetate solution was dried and concentrated, and then the obtained residue was purified by silica gel column chromatography (CHCl₃/ethyl acetate=2/1) to obtain C6-FE-21. In acetonitrile (2 mL) in the presence of diisopropylethylamine (11 µL), C6-FE-21 (10 mg) and TSTU (11.5 mg) were reacted for two hours at 80°C. When the residue obtained by distilling away the reaction solution was dissolved in ethyl acetate, washed with water, dried, and then purified by silica gel column chromatography (CHCl₃/ethyl acetate=2/1), an FE-21-C6-succinimide derivative was obtained. In the present invention, this was used as the L-703,717 succinimide derivative. The process of L-703,717 succinimide derivative synthesis is shown below.

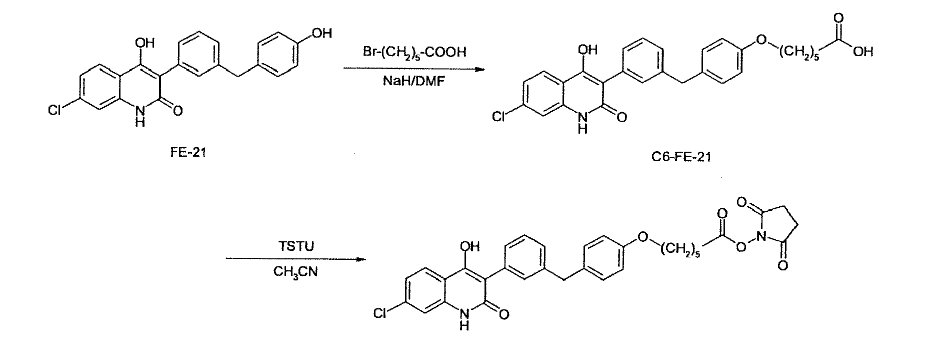

### Linking the [C]K004K peptide and [¹⁸F]SFB

1 mg of the K004K peptide was dissolved in 100 µL of DMF, and the pH was adjusted to 7 to 8 by adding triethylamine (TEA). The total amount of the prepared peptide solution was added to dried [¹⁸F]SFB, and after dissolution, this was reacted at room temperature for 20 to 30 minutes. High performance liquid chromatography (HPLC) was used for purification. The peak fraction eluted at approximately 8.5 minutes from a YMC J'sphere column (10 x 250 mm) by isocratic elution using 23% acetonitrile with 0.1% trifluoroacetic acid (TFA) at 5 mL/min was collected. 20 µL of Tween80 was added to the collected peak fraction, and this was then concentrated to 300 to 400 µL using a rotary evaporator to prepare an injection sample.

### Linking the [C]K004K peptide, [¹⁸F]SFB, and the L-703,717 succinimide derivative

0.6 mg of the K004K peptide was dissolved in 100 µL of DMF, and the pH was adjusted to 7 to 8 by TEA addition. The total amount of the prepared peptide solution was added to dried [¹⁸F]SFB, and after dissolution, this was reacted at room temperature for 20 to 30 minutes. Next, a total volume of 25 µL of DMF solution containing 0.3 µg of the L-703,717 succinimide derivative was added, and this was reacted at room temperature for 20 to 30 minutes. HPLC was used for purification, and the peak fraction eluted at approximately 19 minutes from a YMC J' sphere column (10 x 250 mm) by isocratic elution using 38% acetonitrile with 0.1% TFA at 5 mL/min was collected. 30 µL of Tween80 was added to the collected peak fraction, and then, this was concentrated to 300 to 400 µL using a rotary evaporator to prepare an injection sample.

The above-described linking procedure is described below:
[¹⁸F]SFB (dry) 6.4 µCi
↓ K004K 0.6 mg/110 µL DMF, TEA
↓ RT, 27 min
↓ L-703,717, 0.3 mg/25 µL DMF
↓ RT, 33 min
↓ collect 18-20-min RI peak
↓ dry under reduced pressure
0.467 µCi ([¹⁸F]FB)-K004K-(L-703,717)

### Micro-PET Imaging

Sprague-Dawley rats were anesthetized by an intraperitoneal administration of Nembutal, and a [¹⁸F] Fluorobenzoate (FB)-labeled peptide was administered at the right common carotid artery. During the 30 minutes post-administration, brain images were taken by micro-PET imaging.

### Ex vivo Autoradiography (ARG)

Sprague-Dawley rats were anesthetized by an intraperitoneal administration of Nembutal, and a labeled peptide was administered at the right common carotid artery. After the administration, a 30-minute micro-PET scan was completed, and then the brain was removed and frozen using dry ice. 0.2-µm-thick horizontal frozen sections were prepared from the frozen brain, and placed in contact with an imaging plate for 20 to 30 minutes, and then signals were taken up from the imaging plate using BAS5000 to obtain an ARG image.

### [¹³¹I] iodine-labeling of [C]C004CY

Iodination reaction was carried out using the Chloramine-T method.

After mixing 15 µL of 10 mM [C]C004CY and 5 µL of [¹³¹I]NaI (100 to 200 µCi) with 20 µL of 0.2 M phosphate buffer, 20 µL of 15 mM Chloramine-T dissolved in 0.1 M phosphate buffer was added, and after stirring, this was reacted for 30 seconds at room temperature. The reaction solution was immediately subjected to HPLC purification. The peak fraction eluted at approximately 16 minutes from an Inertsil ODS-2 column (4.6 x 150 mm) by isocratic elution using 20% acetonitrile with 0.1 % trifluoroacetic acid (TFA) at 1 mL/min was collected. 5 µL of Tween80 was added to the collected peak fraction, and this was then freeze-dried and re-dissolved in physiological saline solution to prepare an injection sample.

### In vivo metabolism test

Labeled peptides ([¹³¹I]-labeled peptide: approximately 10 µCi; [¹⁸F]FB-labeled peptide: approximately 100 µCi) were administered to FVB/NJcl mouse tail vein, and one minute or ten minutes after the administration, the blood and brain samples were collected. 1 mL of methanol was added to 200 µL of the blood, and this was stirred vigorously. 2 mL of methanol was added to the brain, and this was homogenized using a Potter Homogenizer. The blood and brain homogenates were centrifuged, and the supernatants were used as tissue extract solutions to collect peptide components. The extracted solutions were developed by thin layer chromatography (TLC) (solvent: butanol: acetic acid: water=4:1:2), and the presence or absence of metabolite spots was examined.

### In vitro metabolism

The following protocol was carried out.
Blood-removed tissue or blood
↓ x 5 vol. PBS
↓ homogenize
↓ dispense in 400-µL aliquots
↓ pre-incubate for 5 minutes at 37°C
↓ add 20 µL of [C]C004CY(¹³¹I)
↓ incubate at 37°C
↓ add 1 mL of MeOH
↓ vortex
↓ 15,000 rpm, 10 min
↓ Sup.
↓ TLC

### [Example 1] Metabolism tests using RI-labeled peptides

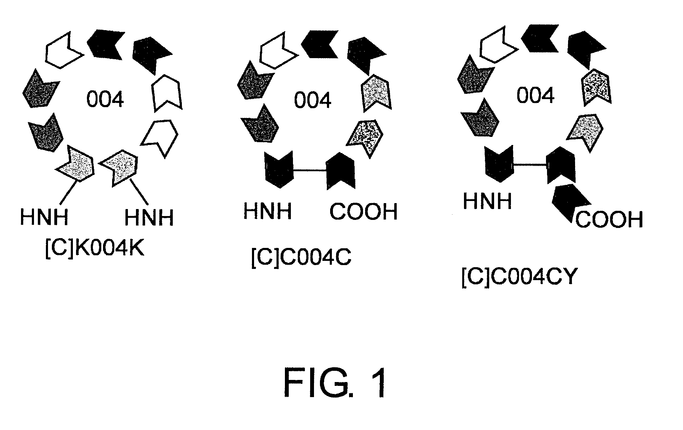

In order for brain-localizing peptides to exhibit their activity, the brain-localizing amino acid motif needs to maintain a cyclic structure. The cyclization may be accomplished by an S-S linkage between the SH groups of cysteines, or an amide bond (peptide bond) formed between the N-terminal α-amino group and C-terminal carboxyl group. The [C]C004CY peptide has a sequence in which the brain-localizing amino acid sequence of SEQ ID NO: 4 is placed between two cysteines (C), and is cyclized by an S-S linkage between the SH groups of cysteines, and carries an added tyrosine (Y) at the C terminus that can be used for iodine labeling. [C] indicates a cyclic structure. On the other hand, the [C]K004K peptide has lysines (K) at both ends of the same brain-localizing amino acid sequence, and is cyclized by the formation of an amide bond (peptide bond) between the N-terminal α-amino group and C-terminal carboxyl group. They are shown in schematic diagrams (Fig. 1).

As a method of introducing a positron nuclide into an ordinary peptide, the method of [¹⁸F] fluorobenzoyl ([¹⁸F]FB)-ation of a free amino group on the peptide is common. This is accomplished easily by reacting an active ester of [¹⁸F]FB ([¹⁸F]SFB) with a peptide carrying an amino group, and allows introduction of the ¹⁸F atom which has a relatively long lifespan (half-life of approximately 110 minutes) for a positron nuclide. In the [C]C004CY peptide, the N-terminal amino group is the only free amino group that can be used for this reaction. On the other hand, if there is yet another free amino group on this peptide, a ligand can be attached to that amino group.

Therefore, the tyrosine (Y) of [C]C004CY was deleted, the cysteines (C) at both ends were replaced with lysines (K) carrying an ε-amino group, and a peptide having two amino groups was synthesized. For the cyclization of this lysine (K)-substituted peptide, an amide bond between N- and C-termini may be suitable as a method for efficient and low-cost production without affecting the fundamental side-chain structure necessary for brain-localizing activity. Thus, by forming an amide bond between the N and C termini of the lysine (K)-substituted peptide, [C]K004K maintaining the cyclic structure essential for brain-localizing activity was prepared. The schematic diagrams of [C]C004CY and [C]K004K are shown in Fig. 1.

In order for brain function imaging to be carried out by attaching [¹⁸F]SFB and the ligand respectively to the brain-targeting peptide, the peptide bridging the labeled molecule and the ligand must not be degraded until it reaches the brain after intravascular injection. To examine whether the basic structures of brain-targeting peptides, [C]C004CY and [C]K004K, are degraded *in vivo*, an *in vivo* metabolism test was performed as follows. Both radiolabeled peptides were administered to mice, and then blood and brain samples were collected one and ten minutes after administration. Peptide components were extracted using methanol, and the extracts were developed by thin layer chromatography (TLC).

[C]C004CY[¹³¹I] produced by [¹³¹I] iodinating the phenyl group of tyrosine (Y) in [C]C004CY by the chloramine-T method was used. Approximately 11.5 µCi of [C]C004CY[¹³¹I] was administered to FVB/NJcl mouse tail vein, then blood and brain samples were collected one and ten minutes after administration, and the labeled peptide components were extracted using methanol. The extracts were analyzed by TLC, and the spot patterns of various tissue extracts and [C]C004CY[¹³¹I] used for the administration were compared.

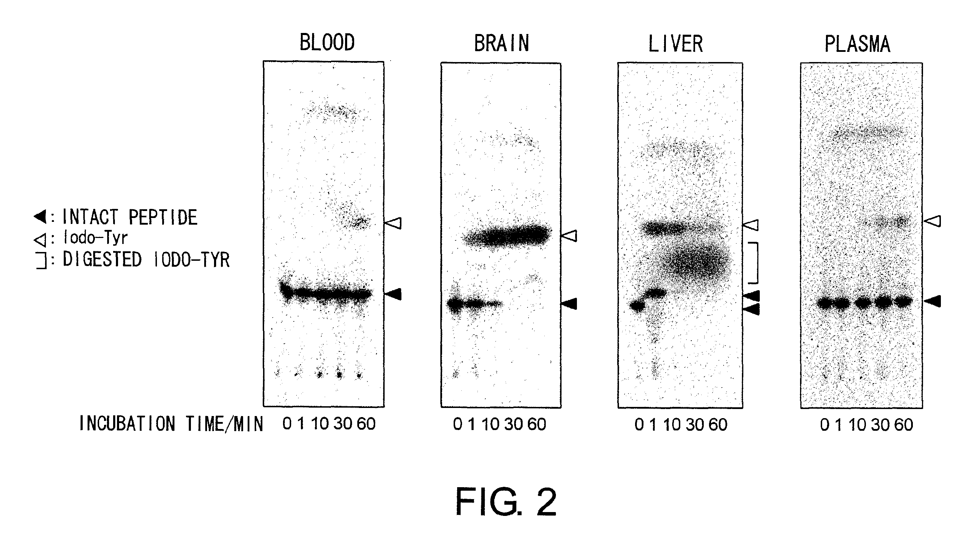

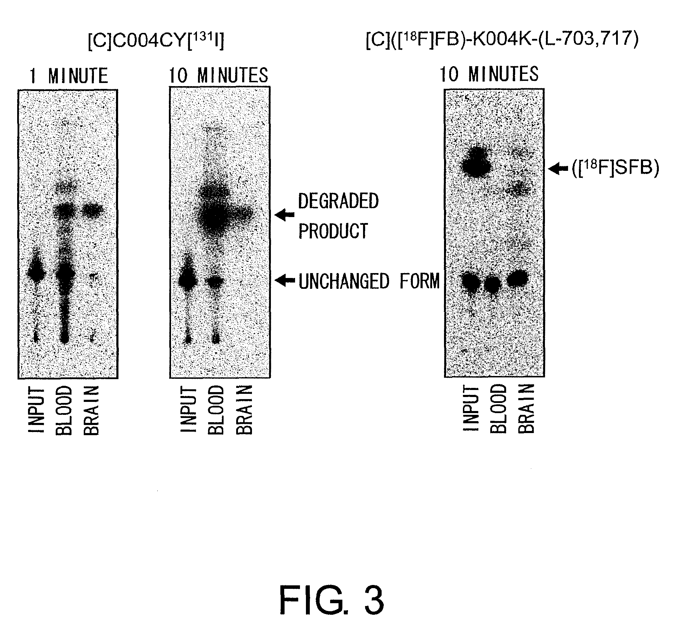

As a result, in mouse blood, spots other than that of [C]C004CY[¹³¹I] were detected one minute after administration indicating that degradation had already started, and ten minutes later, [C]C004CY[¹³¹I] was found to be mostly degraded. [¹³¹I]-labeled forms detected in the brain were also mostly degraded products (Fig. 3). Investigation of how [C]C004CY is metabolized *in vivo* by an *in vitro* metabolism test of [C]C004CY[¹³¹I] showed that it was relatively stable when incubated with blood, but incubation with a brain or liver homogenate led to rapid degradation and [¹³¹I]iodotyrosine was found to dissociate (Fig. 2 and Table 2). According to the above, it is speculated that [C]C004CY[¹³¹I] is stable in blood but when it translocates into peripheral tissues including the brain, it is degraded in the tissues and releases [¹³¹I]iodotyrosine which circulates in blood. Since the degraded [¹³¹I]iodotyrosine is taken up into the cerebral parenchyma *via* an aromatic amino acid transporter, when tyrosine is labeled in this manner, it is difficult to measure the brain localization of the unchanged peptides by radioactivity.

**Table 2**

| | **INTENSITY OF INTACT PEPTIDE (%)** | | | |
|---|---|---|---|---|
| **INCUBATION TIME/MIN** | **BLOOD** | **BRAIN** | **LIVER** | **PLASMA** |
| 0 | 100.0% | 100.0% | 100.0% | 100.0% |
| 1 | 100.0% | 75.9% | 60.2% | 100.0% |
| 10 | 100.0% | 11.2% | 5.7% | 100.0% |
| 30 | 100.0% | 0.0% | 0.0% | 91.0% |
| 60 | 95.2% | 0.0% | 0.0% | 87.5% |

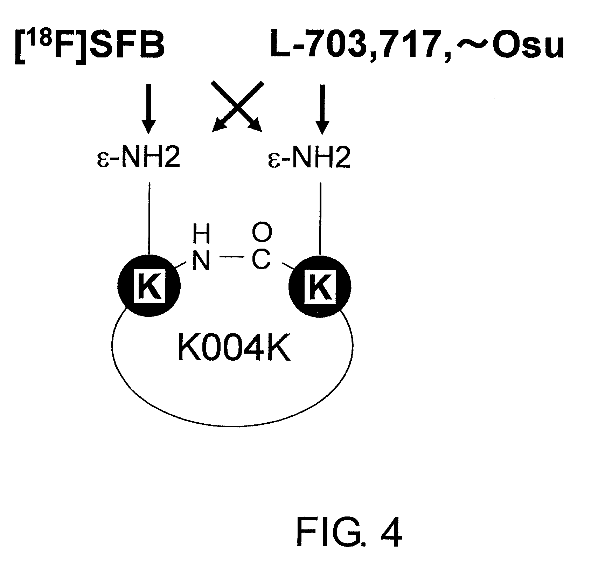

With regard to [C]K004K, [C]([¹⁸F]FB)-K004K-(L-703,717) was synthesized by reacting [C]K004K first with [¹⁸F]SFB, followed by reacting with the succinimide derivative of L-703,717, in which L-703,717 is a ligand for the N-methyl-D-aspartate receptor (a type of cranial nerve receptor) and has low brain-localizing activity. A schematic diagram of the synthesis is shown in Fig. 4.

Approximately 130 µCi of [C]([¹⁸F]FB)-K004K-(L-703,717) was administered to FVB/NJcl mouse tail vein, and blood and brain samples collected ten minutes after the administration were subjected to TLC analysis. As a result, since spots of degraded products of [C]([¹⁸F]FB)-K004K-(L-703,717) were hardly detected in the blood and brain samples, the [C]K004K peptide was confirmed to be stable *in vivo* (Fig. 3).

### [Example 2] In vitro metabolism test

Results of the *in vivo* metabolism test showed that the [C]K004K peptide is more stable *in vivo* compared to [C]C004CY. To compare the degree of improved stability observed in [C]K004K, an experiment system that uses a fluorescent pigment as a tracer instead of an isotope labeling for *in vitro* evaluation using a mouse liver homogenate was produced.

The technique is briefly indicated below.
Add two parts of Hanks' solution to one part of mouse liver, and homogenize
↓
Centrifuge and collect supernatant
↓
Add five parts of Hanks' solution (liver extract solution)
↓
Add 5 µL of peptide to 245 µL of liver extract solution
↓
Incubate at 37°C
↓
Take 50 µL of sample 0, 5, 30, 60, and 120 minutes after incubation
↓
Add 200 µL of 70% ethanol (pre-cooled at -30°C) and then vortex
↓
Add 500 µL of chloroform (pre-cooled at -30°C) and then vigorously vortex
↓
Centrifuge and collect supernatant
↓
Add an equivalent amount of DMF and then vortex
↓
Centrifuge and collect supernatant
↓
HPLC analysis using Co-sense

The peptides that were used were as follows, respectively.
[C](Flu)-C004C: A peptide cyclized by adding C to both ends of the 004 sequence and forming a disulfide bond between them was labeled with fluorescein at its N-terminus.
[C](Flu)-K004C: A peptide cyclized by adding K to the N terminus and C to the C terminus of the 004 sequence and forming a peptide bond between the N- and C-termini was labeled with fluorescein at its N terminus.
[C](Flu)-K004K: A peptide cyclized by adding K to both ends of the 004 sequence and forming a peptide bond between the N- and C-termini was labeled with fluorescein at its N terminus.
[C](Flu)-C004CYK: A peptide cyclized by adding C to the N terminus and CYK to the C terminus of the 004 sequence and forming a disulfide bond between the C's was labeled with fluorescein at its N terminus.
[C]C004CYK (Flu): A peptide cyclized by adding C to the N terminus and CYK to the C terminus of the 004 sequence and forming a disulfide bond between the C's was labeled with fluorescein at its C-terminal K-side chain.

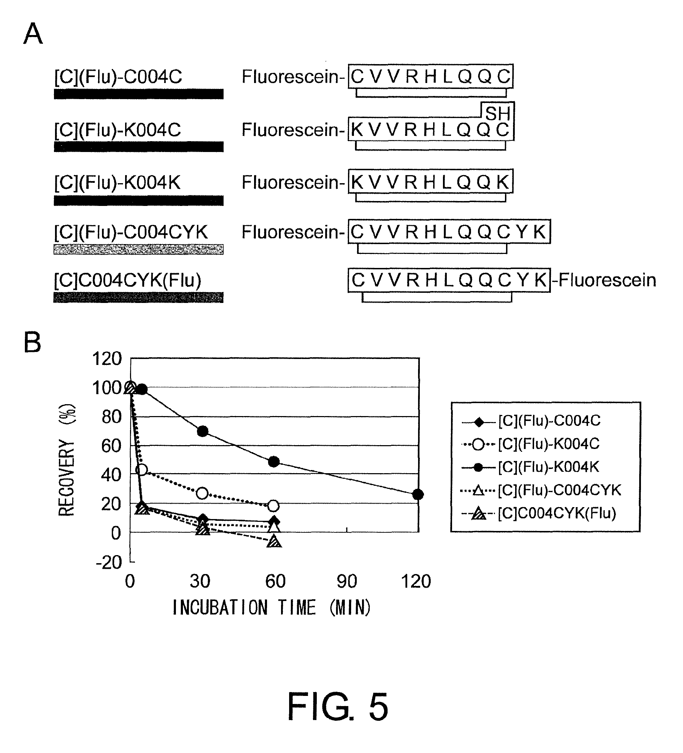

As a result, [C](Flu)-K004K had the strongest resistance to degradation compared to any of the other peptides that had the same sequences except at the ends, and while the residual ratio of most peptides was 10% or less after a 60-minute incubation, approximately 50% of this peptide remained in the unchanged form, and this corresponds well with the *in vivo* test results. K004C produced by homodetic cyclization as in K004K decreased to 40% in five minutes (Fig. 5).

While [C]K004K was produced for the purpose of conferring multiple amino groups to a brain-targeting peptide, its resistance to degradation *in vivo* was dramatically improved as a result. This result is thought to be caused by the peptide becoming less prone to attack by exopeptidases and such due to the disappearance of the amino terminus, and the absence of highly reactive side chains like the thiol group of cysteine (C) leading to fewer non-specific interactions with *in vivo* factors.

### [Example 3] Translocation of peptides to the cerebral parenchyma

Next, to verify the translocation of [C]K004K into the cerebral parenchyma, [C]([¹⁸F]FB)-K004K was synthesized by positron-labeling the K004K peptide through reaction of ¹⁸F-labeled SFB with the amino group of [C]K004K, and the *in vivo* brain-localizing characteristic was examined.

[C]([¹⁸F]FB)-K004K was synthesized by adding [¹⁸F]SFB to [C]K004K and letting this react at room temperature. Approximately 0.57 mCi/300 µL of the synthesized [C]([¹⁸F]FB)-K004K was administered to the right common carotid artery of Sprague-Dawley rats, and they were subjected to micro-PET scanning for 30 minutes to obtain bioimages of the brain.

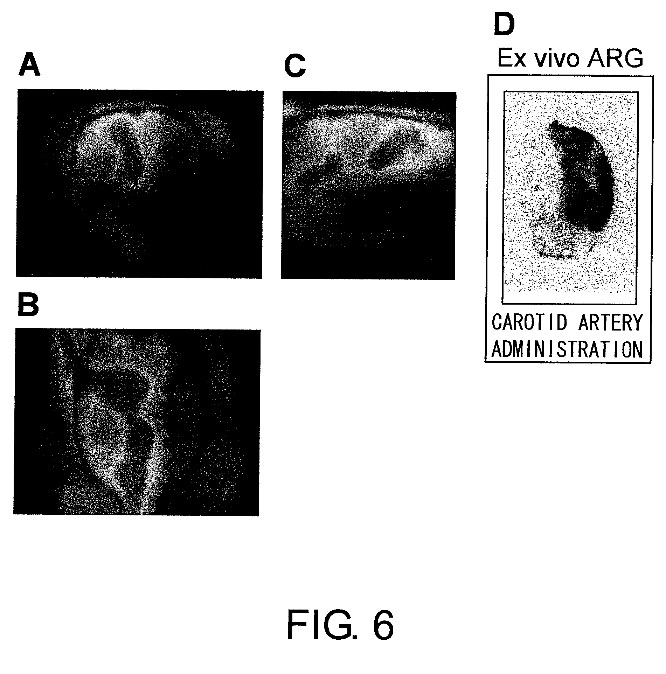

As a result, very strong signals were detected only on the side of the brain receiving the administration, and hardly any signals were detected on the other side of the brain and in the cerebellum (Fig. 6A, B, and C).

Furthermore, the scanned brain was removed and subjected to *ex vivo* autoradiography (ARG). Protocols for PET and ARG in carotid artery administration are briefly described below.
approximately 550 µCi/300 µL of [C]([¹⁸F]FB)-K004K
↓ administration at the right common carotid artery of rats
PET imaging (30 min)
↓
brain removal
↓ preparation of frozen sections
ARG

As a result, very strong diffuse signals were detected in the thalamus, hippocampus, and parahippocampal gyrus on the administered side, but hardly any signals were detected on the other side of the brain and in the cerebellum (Fig. 6D). This showed that [C]([¹⁸F]FB)-K004K quickly translocates to the cerebral parenchyma after administration at the carotid artery, and does not easily translocate to the striatum.

Furthermore, SFB-labeled K004K was administered to the mouse tail vein. Protocols for PET and ARG in mouse tail vein administration are briefly described below: i.v. administration to mice,
80 µCi/300 µL saline, 2.5% Evans Blue,
scan for 30 minutes.

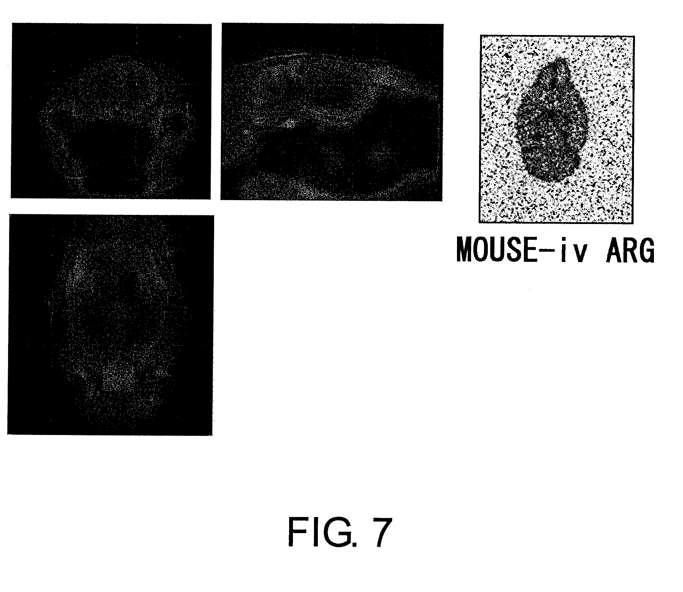

As a result of PET, a certain level of signals was observed in the brain, but since the signals from the face were too strong, signals from the brain seemed to be missing. Since *ex vivo* ARG shows a certain level of signals in the cerebral parenchyma, it is believed that a certain level of the peptide enters the brain even by injection into the tail vein (Fig. 7).

### [Example 4] Translocation of the ligand into the cerebral parenchyma

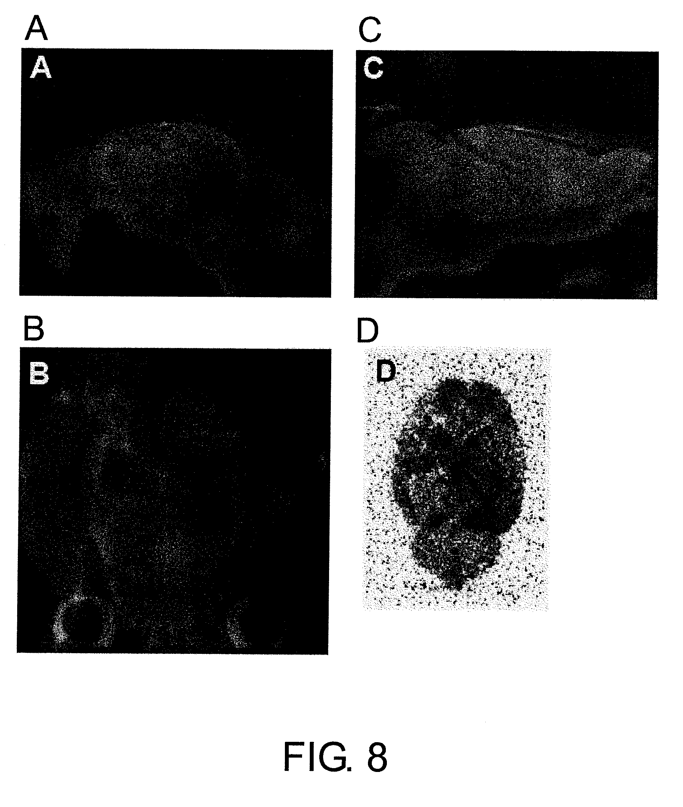

Since translocation of [C]K004K to the cerebral parenchyma was confirmed, whether [C]K004K can transport a PET ligand into the brain was then verified by carrying out PET imaging of [C]([¹⁸F]FB)-K004K-(L-703,717) (Fig. 8). [C]([¹⁸F]FB)-K004K-(L-703,717) was synthesized by reacting [C]K004K with [¹⁸F]SFB, followed by a continued reaction with the L-703,717 succinimide derivative. Approximately 0.33 µCi/300 µL of the synthesized [C]([¹⁸F]FB)-K004K was administered at the right common carotid artery of Sprague-Dawley rats, and they were subjected to micro-PET scanning for 30 minutes to obtain bioimages of the brain.

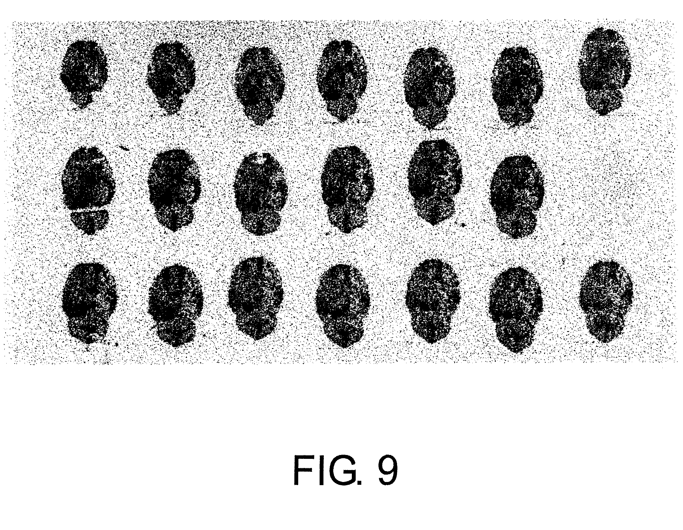

As a result, strong signals were detected on the side of administration, as well as on the other side of the brain and in the cerebellum. The brain was removed after the PET scan and subjected to *ex vivo* ARG (Fig. 8D and Fig. 9).

Verification protocol using the above-mentioned micro-PET and *ex vivo* ARG is described below.
333 µCi/300 µL saline (ca 7.5% Tween80)
administration to the rat common carotid artery
↓
PET scan for 30 minutes
↓
*ex vivo* ARG
IP contact for 30 minutes

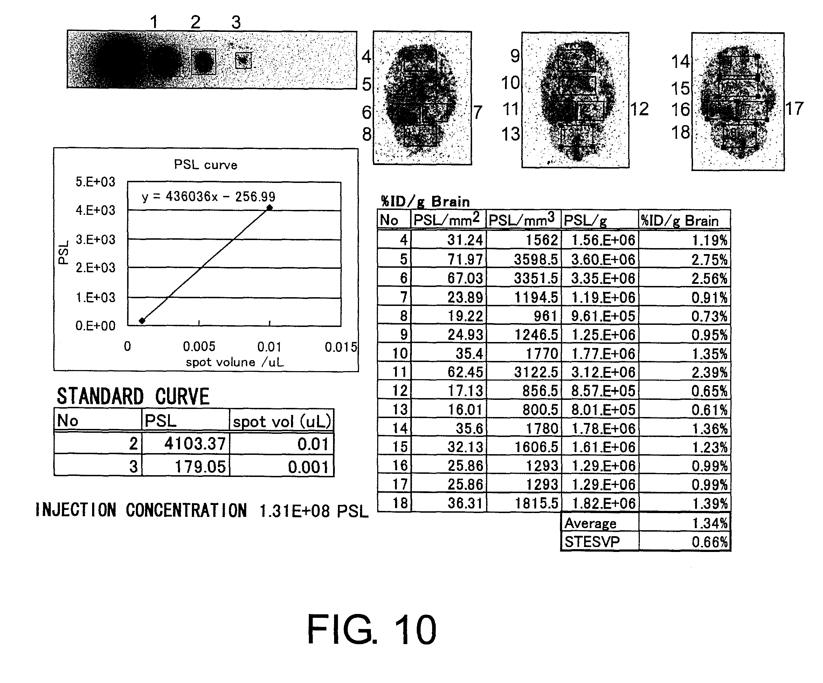

As a result, diffuse signals were detected throughout the whole brain including the other side and the cerebellum. When the brain-localizing ratio was calculated from the signal intensities (PSL) of the ARG image, a high brain-localizing ratio of 1.34% ± 0.66% ID/g Brain was indicated. Figures and tables relating to the PSL curve and brain-localizing ratio determined by ARG are shown together in Fig. 10.

From these findings, when administered to the carotid artery, [C]K004K was found to translocate a ligand to the cerebral parenchyma.

### Industrial Applicability

The present invention provides brain-localizing polypeptides, which while maintaining brain-localizing activity, are not easily degraded *in vivo* and can link to multiple molecules. Polypeptides of the present invention were found to be able to transport even PET ligands which have low brain-localizing activity to the cerebral parenchyma. Furthermore, since these improved brain-targeting peptides have two binding moieties, if a positron nuclide is introduced to one, various PET ligands can be introduced to the other under similar reaction conditions, and this makes them highly versatile. Additionally, since a plurality of molecules such as a therapeutic agent and polyethylene glycol can be introduced, more effective treatment can be expected in treating brain diseases.

The present invention provides possibilities for the peptides to act as tools for creating new possibilities for many PET ligands and pharmaceutical agents of which development has been abandoned due to their inability to pass through the blood brain barrier.

Polypeptides of the present invention are, for example, polypeptides that form a crosslink between compounds A and B having two different functions, and confer brain-localizing characteristic to the whole molecule formed by the cross-linking. For example, they are molecules that combine molecule A which exhibits a function of binding to a receptor in the brain *ex vivo* but does not enter the brain *in vivo,* with molecule B labeled with a PET nuclide, thereby enabling diagnosis of brain receptor functions which would have been otherwise completely impossible. Great many useful combinations of two molecules A and B similar to this example can be considered.

Since ¹⁸F emits positrons, this labeling can be used to evaluate the brain-localizing activity of peptides linked to a variety of compounds by PET imaging in addition to radiography. When the variety of compounds are ligands that bind to molecules playing important roles in biological functions such as receptors in the brain, functions of the biologically functional molecules can be examined, and one can screen for substances that inhibit the binding between the biologically functional molecules and their ligands.

## Claims

1. A cyclized polypeptide comprising a multivalent binding moiety, which is a cyclized polypeptide comprising a brain-localizing motif sequence and at least two lysine residues.

2. The cyclized polypeptide of claim 1, comprising two adjacent lysine residues.

3. The cyclized polypeptide of claim 1 or 2, wherein metabolic stability is improved.

4. The cyclized polypeptide of any one of claims 1 to 3, wherein a PET nuclide-labeled molecule is attached to the ε-amino group of lysine, and/or a ligand molecule of a brain receptor is attached to the ε-amino group of another lysine.

5. A carrier molecule for brain localization comprising the cyclized polypeptide of any one of claims 1 to 3 as an active ingredient.

6. A brain-localizing pharmaceutical agent, wherein a pharmaceutical agent is attached to the ε-amino group of a lysine residue of the cyclized polypeptide of any one of claims 1 to 3.

7. A reagent for PET examination, which comprises the cyclized polypeptide of claim 4 as an active ingredient.

8. A kit for producing a brain-localizing pharmaceutical agent, comprising at least the following substances as components:
(a) the cyclized polypeptide of any one of claims 1 to 3; and
(b) a pharmaceutical agent.

9. A kit for PET examination, which comprises at least the following substances as components:
(a) a cyclized polypeptide of any one of claims 1 to 3; and
(b) a molecule labeled with a PET nuclide.

10. A method for producing a cyclized brain-localizing polypeptide comprising a multivalent binding moiety, which comprises the step of forming an amide bond between the two ends of a polypeptide comprising lysines at both ends and a brain-localizing motif sequence.

11. A method for producing a brain-localizing polypeptide with improved metabolic stability, which comprises the step of forming an amide bond between the two ends of a polypeptide comprising lysines at both ends and a brain-localizing motif sequence.

12. A method for producing a brain-localizing pharmaceutical agent, comprising the step of attaching the ε-amino group of a lysine of the cyclized polypeptide of any one of claims 1 to 3 to a pharmaceutical agent.

13. A method for producing a regent for PET examination, comprising the step of attaching the ε-amino group of a lysine of the cyclized polypeptide of any one of claims 1 to 3 to a molecule labeled with a PET nuclide.

14. A method for producing a brain-localizing ligand molecule, comprising the step of attaching the ε-amino group of a lysine of the cyclized polypeptide of any one of claims 1 to 3 to a ligand molecule for a brain receptor.

15. A method for cyclizing a brain-localizing polypeptide in a state that carries a multivalent binding moiety, comprising the step of forming an amide bond between the two ends of a polypeptide comprising lysines at both ends and a brain-localizing motif sequence.

16. A method of improving metabolic stability of a brain-localizing polypeptide, comprising the step of forming an amide bond between the two ends of a polypeptide comprising lysines at both ends and a brain-localizing motif sequence.


SEQUENCE LISTING

<110>  Proteus Sciences Co., Ltd.
       NATIONAL INSTITUTE OF RADIOLOGICAL SCIENCES

<120>  BRAIN-MIGRATING POLYPEPTIDE HAVING POLYVALENT BOND
       AND IMPROVED METABOLIC STABILITY

<130>  EP66957FZpau

<140>  08 720 933.4
<141>  2008-02-27

<150>  PCT/JP2008/053385
<151>  2008-02-27

<150>  JP 2007-054260
<151>  2007-03-05

<160>  24

<170>  PatentIn version 3.4

<210>  1
<211>  9
<212>  PRT
<213>  Artificial

<220>
<223>  an artificially synthesized sequence

<400>  1

Cys Ser Asn Leu Leu Ser Arg His Cys
1               5


<210>  2
<211>  9
<212>  PRT
<213>  Artificial

<220>
<223>  an artificially synthesized sequence

<400>  2

Cys Ser Leu Asn Thr Arg Ser Gln Cys
1               5


<210>  3
<211>  9
<212>  PRT
<213>  Artificial

<220>
<223>  an artificially synthesized sequence

<400>  3

Cys Val Ala Pro Ser Arg Ala Thr Cys
1               5


<210>  4
<211>  9
<212>  PRT

<213>  Artificial

<220>
<223>  an artificially synthesized sequence

<400>  4

Cys Val Val Arg His Leu Gln Gln Cys
1               5


<210>  5
<211>  9
<212>  PRT
<213>  Artificial

<220>
<223>  an artificially synthesized sequence

<400>  5

Cys Val Leu Arg His Leu Gln Gln Cys
1               5


<210>  6
<211>  9
<212>  PRT
<213>  Artificial

<220>
<223>  an artificially synthesized sequence

<400>  6

Cys Arg Gln Leu Val Gln Val His Cys
1               5


<210>  7
<211>  9
<212>  PRT
<213>  Artificial

<220>
<223>  an artificially synthesized sequence

<400>  7

Cys Gly Pro Leu Lys Thr Ser Ala Cys
1               5


<210>  8
<211>  9
<212>  PRT
<213>  Artificial

<220>
<223>  an artificially synthesized sequence

<400>  8

Cys Leu Lys Pro Gly Pro Lys His Cys
1               5


<210>  9

<211>  9
<212>  PRT
<213>  Artificial

<220>
<223>  an artificially synthesized sequence

<400>  9

Cys Arg Ser Pro Gln Pro Ala Val Cys
1               5


<210>  10
<211>  9
<212>  PRT
<213>  Artificial

<220>
<223>  an artificially synthesized sequence

<400>  10

Cys Asn Pro Leu Ser Pro Arg Ser Cys
1               5


<210>  11
<211>  9
<212>  PRT
<213>  Artificial

<220>
<223>  an artificially synthesized sequence

<400>  11

Cys Pro Ala Gly Ala Val Lys Ser Cys
1               5


<210>  12
<211>  9
<212>  PRT
<213>  Artificial

<220>
<223>  an artificially synthesized sequence

<400>  12

Cys Pro Ala Gly Ala Leu Lys Ser Cys
1               5


<210>  13
<211>  7
<212>  PRT
<213>  Artificial

<220>
<223>  an artificially synthesized sequence

<400>  13

Ser Asn Leu Leu Ser Arg His
1               5

<210>  14
<211>  7
<212>  PRT
<213>  Artificial

<220>
<223>  an artificially synthesized sequence

<400>  14

Ser Leu Asn Thr Arg Ser Gln
1               5


<210>  15
<211>  7
<212>  PRT
<213>  Artificial

<220>
<223>  an artificially synthesized sequence

<400>  15

Val Ala Pro Ser Arg Ala Thr
1               5


<210>  16
<211>  7
<212>  PRT
<213>  Artificial

<220>
<223>  an artificially synthesized sequence

<400>  16

Val Val Arg His Leu Gln Gln
1               5


<210>  17
<211>  7
<212>  PRT
<213>  Artificial

<220>
<223>  an artificially synthesized sequence

<400>  17

Val Leu Arg His Leu Gln Gln
1               5


<210>  18
<211>  7
<212>  PRT
<213>  Artificial

<220>
<223>  an artificially synthesized sequence

<400>  18

Arg Gln Leu Val Gln Val His

1                   5


<210>  19
<211>  7
<212>  PRT
<213>  Artificial

<220>
<223>  an artificially synthesized sequence

<400>  19

Gly Pro Leu Lys Thr Ser Ala
1               5


<210>  20
<211>  7
<212>  PRT
<213>  Artificial

<220>
<223>  an artificially synthesized sequence

<400>  20

Leu Lys Pro Gly Pro Lys His
1               5


<210>  21
<211>  7
<212>  PRT
<213>  Artificial

<220>
<223>  an artificially synthesized sequence

<400>  21

Arg Ser Pro Gln Pro Ala Val
1               5


<210>  22
<211>  7
<212>  PRT
<213>  Artificial

<220>
<223>  an artificially synthesized sequence

<400>  22

Asn Pro Leu Ser Pro Arg Ser
1               5


<210>  23
<211>  7
<212>  PRT
<213>  Artificial

<220>
<223>  an artificially synthesized sequence

<400>  23

[?]

Pro Ala Gly Ala Val Lys Ser
1               5

<210>  24
<211>  7
<212>  PRT
<213>  Artificial

<220>
<223>  an artificially synthesized sequence

<400>  24

Pro Ala Gly Ala Leu Lys Ser
1               5